# EUROPEAN PATENT APPLICATION

(11) **EP 0 628 323 A1**
(43) Date of publication of application: **14.12.1994**
(21) Application number: 94201603.1
(22) Date of filing: 06.06.1994
(51) Int. Cl.: A61M 25/01

(54) **Controllably bendable catheter**

(30) Priority: 11.06.1993 NL 9301018
(71) Applicant: CORDIS EUROPA N.V., NL-9301 LJ Roden (NL)
(72) Inventor: Van Erp, Wilhelmus Petrus Martinus Maria, NL-9351 KS Leek (NL)
(74) Representative: 't Jong, Bastiaan Jacobus

(57) **Abstract**

The invention relates to a catheter comprising a tube-like basic body with a distal end and a proximal end provided with a connecting means, at least one continuous channel and with a flexible part at the distal end.
In the flexible end-section a pull wire, extending through the channel to the outside of the proximal end, is attached eccentrically.
The deflection of the end-section can be varied with the aid of a sliding wire advanced further or less far in a second channel in the end-section.

## Description

The invention relates to a catheter, in particular a catheter for angiographic purposes, comprising a tube-like basic body with a distal end and a proximal end provided with a connecting piece. In the catheter a pull wire is arranged, such that when the pull wire is pulled, the end section of the catheter bends in a controlled manner.

A catheter of this kind is known from EP-A-0 361 314.

With the invention as characterized in claim 1, an improved steerability of the catheter is achieved. The comparatively stiff sliding wire renders the end-section into which it extends stiff, so that primarily only that part of the end-section into which the sliding wire does not extend, bends. By advancing the sliding wire more or less far into its channel, the shape of the deflecting part can be adjusted.

An advantageous embodiment is characterized in claim 3. The end-section and the basic body of the catheter may be extrusion profiles with a number of channels extending over the entire length.

To allow proper and careful manipulation of the catheter according to the invention, the measure as set out in claim 4 is preferably applied. When in use the physician can hold the handle in one hand and operate the control means with a few fingers of the same hand.

A very suitable embodiment is characterized in claim 5. The rotatable case can be operated with the thumb and index finger, while the handle is held with the other fingers. Transmission of the rotational movement of the case to the pull wire is effected in a very simple and reliable manner, also keeping the manufacturing costs of the contraption relatively low.

To achieve adequate control of the sliding wire as well, the measure as set out in claim 6 is preferably applied. Adjustment of the sliding wire is achieved with the aid of the second control means after which the deflection can be further determined by using the first control means.

The invention will be explained in greater detail in the following description with reference to the examples of the embodiments as shown in the drawings.
- **Fig. 1**: represents a catheter according to the invention in the position of use.
- **Fig. 2**: shows the end-section of a catheter according to a first embodiment of the invention in a partly longitudinal cross-sectional view.
- **Fig. 3**: shows a view of another embodiment corresponding to fig. 2.
- **Fig. 4**: represents the catheter of fig. 3 in another position of use.
- **Fig. 5**: represents a partly broken away perspective view of a control handle of a catheter according to the invention.

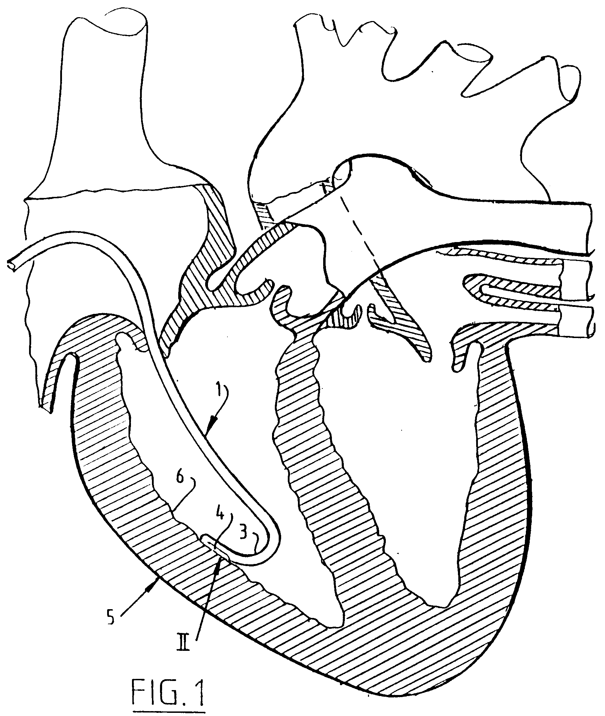

As fig. 1 shows, a catheter 1 according to the invention can be advanced in the usual manner through a vein to the right ventricle in order to be positioned firmly against the wall 6 of the right ventricle of the heart 5.

The catheter 1 comprises a basic body with a proximal end, in the usual manner provided with a connecting piece not shown here remaining outside the body, and a distal end comprising a flexible end-section 3.

With this embodiment the wall of the flexible end-section is provided with a strip-like electrode extending in the longitudinal direction of the catheter, such as a heating element.

The catheter 1 is, according to the invention, manufactured in such a way that its end-section 3 can be bend reliably in a controlled fashion in order to place electrode 4 firmly against the wall 6. When the electrode 4 is a heating element, as described above, a certain section of the wall 6 can be heated in this way and consequently the tissue disturbed when treating certain types of cardiac arrhythmias.

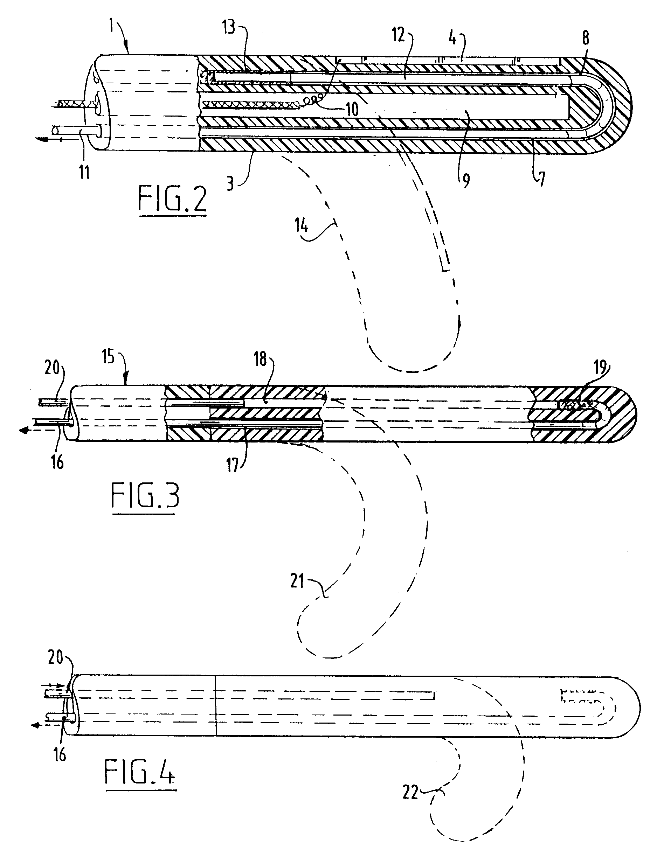

As fig. 2 shows in greater detail, the catheter 1 is provided with an eccentrically positioned channel 7, in which a pull wire 11 has been incorporated. This pull wire has been fixed eccentrically in the end-section 3, by incorporating a doubled up end-piece 12 of the pull wire 11 in a cavity 8. The end-piece 12 can be placed against an end wall of the body of the catheter, at the position of the connection with a separate flexible end-section.

The pull wire 11 extends over the full length of the catheter 1 through the channel 7 and ends, close to the proximal end outside the catheter. By pulling pull wire 11, the end-section 3 of the catheter 1 bends in the direction of channel 7, in which the pull wire 11 has been incorporated. The deflection has been illustrated schematically and is indicated with reference numberal 14.

The doubled up end-piece 12 of the pull wire 11 has been secured in channel 8 with glue 13. Another possibility being, that the end-section 3 is a separate end-section of the catheter 1 and that the doubled back end-piece 12 of the pull wire is placed against the end wall of the basic body at the position of the connection between the end-section and the basic body.

Fig. 2 also shows cross-sectionally the strip-like electrode executed as heating element 4 together with a connecting wire 10, running through a central channel 9 in the catheter, to the proximal end where this connecting wire is connected.

In relation to the channel 7, the electrode 4 is placed at the opposite side of the catheter, so that this electrode 4 is always positioned on the outside curve of the bent catheter and consequently can be moved firmly against the desired object.

It is obviously also possible to place the electrode at the opposite side, close to the eccentrically located channel 7 in which case the electrode will always be placed on the inside of the curve.

The catheter 15 of fig. 3 also comprises an eccentrically positioned channel 17 in the end-section of which the pull wire 16 has been incorporated. The end of the pull wire has been doubled up over a distance 19 and engages in a channel 18 which is for the most part situated diametrically opposite the channel 17. Furthermore, the channel 18 extends the entire length of the end-section comprising a movable sliding wire 20, which is comparatively stiff. With the position of the sliding wire 20 as shown in fig. 3 the flexibility of the end-section is not or hardly affected by the sliding wire 20, so that when the pull wire 16 is pulled, a deflection 21 with a comparatively large bending radius is created.

When on the other hand, as shown in fig. 4, the sliding wire 20 is advanced further in the channel 18, a deflection 22 with a much smaller radius will be formed when pulling the pull wire 16, as basically only that part of the end-section will bend in which the sliding wire 20 is not situated.

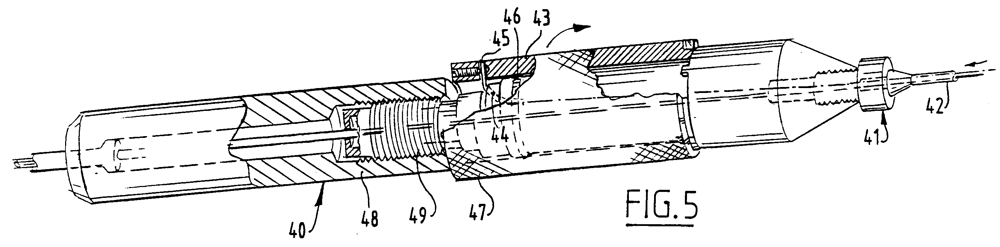

Fig. 5 represents a control means which can be used with a catheter according to the invention. The catheter together with pull wire and if required sliding wire, is connected to the right hand side of the control means 40 by means of a connecting means 41. The catheter 42 is connected firmly to the control means 40.

The control means 40 comprises a handle 48 and a, in relation to the handle 48, rotatable case 43. By means of a threaded joint 49 the handle 48 is screwed onto a basic element allowing the case 43 to rotate. This basic body and the handle comprise a continuous channel through which for example connecting wires for a heating element, a sliding wire, a guide wire, a contrast medium line etc. can be fed. A tangential channel 44 is connected to this central channel through which the pull wire 45 has been threaded. The pull wire 45 is connected to the case 43, so that by turning the case 43 the pull wire 45 is being pulled.

The case 43 is running in bearings on a rubber ring 46, causing friction against rotation of the case 43 to such an extent, that the latter remains fixed in a particular chosen position of rotation. On the outside the case 43 is provided with a milled surface ensuring a good grip.

The physician using the catheter with the control means 40, holds the control means at the handle 48 and can rotate the case 43 with his thumb and index finger. Bending of the end-section can thus be operated in a carefully controlled manner.

## Claims

1. Catheter comprising a tube-like basic body with a distal end and a proximal end provided with a connecting means, at least one continuous channel and with a flexible part at the distal end, wherein a pull wire, extending through the channel to the proximal end, is attached in the flexible end-section.

2. Catheter as claimed in claim 1, wherein in the end-section the channel through which the pull wire extends is eccentrically positioned and the channel receiving the sliding wire is positioned diametrically opposite the channel containing the pull wire.

3. Catheter as claimed in claim 2, wherein the pull wire is doubled back in a hook-shaped fashion at its end and the bent end-piece is received in the channel receiving the sliding wire.

4. Catheter as claimed in one of the previous claims, wherein a control means has been attached to the proximal end comprising a handle and a, in relation to the handle, moveable operating mechanism, wherein the basic body is attached to the handle and the end of the pull wire to the operating mechanism.

5. Catheter as claimed in claim 4, wherein the operating mechanism consists of a case which can be rotated around the handle and the pull wire is threaded into the handle and attached to the case via a tangential channel formed at the position of the case.

6. Catheter as claimed in one of the claims 4 or 5, wherein the control means comprises a second, in relation to the handle, moveable operating mechanism, to which the end of the sliding wire has been attached.
